# EUROPEAN PATENT APPLICATION

(11) **EP 4 748 366 A1**
(43) Date of publication of application: **27.05.2026**
(21) Application number: 24214422.8
(22) Date of filing: 21.11.2024
(51) Int. Cl.: A61K 8/22, A61K 8/46, A61K 8/81, A61Q 11/00, A61K 8/55

(54) **ORAL CARE COMPOSITION**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

A novel dentifrice composition with superior anti-sensitivity and whitening properties is provided. The dentifrice composition comprising
a) cross-linked polyvinylpyrrolidone-hydrogen peroxide complex (cPVP-H₂O₂ complex);
b) sodium tripolyphosphate (STP)
c) at least one solvent, wherein the at least one solvent comprises sodium lauryl sulphate, and
d) at least one desensitising agent, wherein the at least one desensitizing agent comprises potassium nitrate.

## Description

### BACKGROUND

Oral care products with teeth whitening attributes use a variety of active ingredients to remove stains or whiten teeth. There are two main modes of action for whitening products, physical (abrasion to remove discolouration from the surface) and chemical using bleaching chemistry, normally oxygen-based bleaches.

Chemical whitening is typically the more powerful whitening effect. However, this chemistry is hard to stabilise in dentifrice products and can react with other ingredients prior to use by the consumer.

The most commonly used chemical whitening active ingredients are peroxide sources, such as hydrogen peroxide. These products typically contain substantial amounts of the peroxide source, usually up to about 10 percent hydrogen peroxide.

However, in high concentrations, hydrogen peroxide can be irritating to the teeth and gums and, in addition, hydrogen peroxide is an unstable molecule that is prone to decomposition, especially in aqueous environments.

Oral care products typically have other requirements for users. One such requirement is dentinal (hyper) sensitivity. This is a well-known phenomenon; people suffering from dentinal (hyper) sensitivity experience a painful reaction when their teeth are exposed to cold, hot, sweet, drying, tactile and similar stimuli, e.g. when eating ice-cream or brushing their teeth. The painful stimulus is generally attributed to rapid fluid movements within the dentinal tubules which run through the thickness of the dentine to the nerve terminals in the pre- dentine.

Typically, dentifrice products that treat sensitivity do not also utilise whitening chemistry due to the stability requirements of the two different actives.

Accordingly, there is a desire for oral care composition using lower concentrations of a peroxide source, and/or alternative oxidizing agents, that are stable, provide effective teeth whitening, and alleviating dentinal (hyper) sensitivity for a user.

### STATEMENT OF INVENTION

According to the present invention there is provided a dentifrice composition comprising:
a) cross-linked polyvinylpyrrolidone-hydrogen peroxide complex (cPVP-H₂O₂ complex);
b) sodium tripolyphosphate (STP)
c) at least one solvent, wherein the at least one solvent comprises sodium lauryl sulphate, and
d) at least one desensitising agent, wherein the at least one desensitizing agent comprises potassium nitrate.

The applicants have surprisingly found a dentifrice composition that combines superior whitening performance in combination with excellent desensitization properties.

The invention also comprises the use of the composition in combination with a toothbrush to clean teeth.

The invention also features a method for tooth whitening including applying the tooth composition of the present invention to at least one tooth.

As used herein the term "dentifrice" includes any semi-solid preparation in the form of a paste, cream or gel for use in cleaning or treating all, or a portion of, the oral cavity of an individual.

The present inventors have discovered that the dentifrice of the invention provides excellent whitening whilst maintaining sensitivity protection, allowing users to whiten teeth/dentures with confidence.

In more detail it has been found that the dentifrice of the invention may whiten up to 5 shades (when brushing twice daily for 4 weeks). Here with the use of the term shades it will be understood that shades can be determined before and after treatment using any of a number of shade guides, including, e.g., the VITA^{®} (Vita Zahnfabrik H. Rauter GmbH & Co., KG), CHROMASCOP^{®} (Ivoclar Vivadent, Inc.) or BIODENT (Dentsply International) shade guides. Optionally, a shade taking system, e.g., the ShadeEye NCC Dental Chroma Meter, can be employed to determine shade before and/or after treatment.

In the achievement of this whitening effect, it is understood that the peroxide is stabilised within the composition. It is reasoned that this enhanced stability arises (at least in part) due to the complexation with polyvinylpyrrolidone. This has been found to enhance the stability of the peroxide whitening agent.

Additionally, it has been found that with the use of the cross-linked polyvinylpyrrolidone-hydrogen peroxide complex (cPVP-H₂0₂ complex) provides greater retention of the oral care composition to the tooth surface to maximize delivery of an effective concentration of the peroxide whitening agent at the target site.

### DETAILED DESCRIPTION OF THE INVENTION

### Cross-linked polyvinylpyrrolidone-hydrogen peroxide complex

The peroxide source includes a cross-linked polyvinylpyrrolidone-hydrogen peroxide complex (cPVP-H₂O₂ complex). The cPVP-H₂O₂ complex consists of hydrogen peroxide molecules intimately bound to the polyvinylpyrrolidone backbone. The cPVP-H₂O₂ complex is available commercially as a dry powder for incorporation into whitening and oxidizing compositions and typically consists of from about 15 weight percent to about 30 weight percent hydrogen peroxide, for example, about 18 weight percent hydrogen peroxide. A commercial source of the cPVP-H₂O₂ complex that is suitable for use in the present invention, is available commercially as PEROXYDONE XL 10 (Ashland Inc., Covington KY).

The amount of cPVP-H₂O₂ complex is selected to provide from about 0.01 weight percent to about 6 weight percent hydrogen peroxide to the dentifrice composition. For example, the amount of cPVP-H₂O₂ complex in the oral care composition may provide from about 0.01 weight percent to about 5 weight percent hydrogen peroxide, from about 0 01 weight percent to about 4 weight percent hydrogen peroxide, from about 0.5 weight percent to about 4 weight percent hydrogen peroxide, from about 1 weight percent to about 4 weight percent hydrogen peroxide, from about 1.5 weight percent to about 3.5 weight percent hydrogen peroxide and/or from about 2 weight percent to about 3 weight percent hydrogen peroxide in the oral care composition.

The amount of cPVP-H₂O₂ complex in the oral care composition will be understood to relate to the amount of hydrogen peroxide in the cPVP-H₂O₂ complex to provide the preferred amounts of hydrogen peroxide to the dentifrice composition. For PEROXYDONE XL 10 (Ashland Inc., Covington KY) this is understood to be a multiple of about 3-5 of the amounts discussed above.

### Anti-sensitivity

At least one desensitizing agent, including a tubule blocking agent or a nerve desensitizing agent and mixtures thereof, for example as described in WO 02/15809 (Block) is included in a composition according to the invention.

The formulations of the present invention utilise potassium nitrate as the at least one desensitisation agent.

Preferably the desensitizing agent is present in an amount of 0.1 to 10 percent by weight of the composition. The desensitizing agent may be present in an amount of 1 to 9 percent, 2 to 8 percent, 3 to 7 percent, 4 to 6 percent, about 5 percent, by weight of the composition

Potassium nitrate may be the sole desensitisation agent in the composition of the invention.

Alternatively, potassium nitrate may be used in combination with additional desensitisation agents.

Other The desensitizing agent may comprise a strontium salt such as strontium chloride, strontium acetate or strontium nitrate or another potassium salt such as potassium citrate, potassium chloride, potassium bicarbonate, potassium gluconate.

A desensitizing agent may also include a bioactive glass. A particularly preferred bioglass is Novamin, as seen in oral care products manufactured by Haleon.

### Diluent

Preferably the dentifrice comprises a single phase non-aqueous composition comprising at least one solvent.

The at least one solvent comprises glycerol, polyethylene glycol (PEG), propylene glycol (PPG) or a mixture thereof.

Glycerine is also known as glycerol and glycerin. The terms may be used interchangeably in this specification.

It is well known that commercially available glycerine may contain between 0.1-2.0 percent by weight of water which is in association with the glycerine. Typically, this amount is < 0.5 percent and for example between 0.1 - 0.5 percent by weight of the glycerine. This small amount of water is bound to the glycerine and is therefore not available to the other ingredients. The skilled person would still consider a composition containing glycerine as being non-aqueous.

Preferably the at least one solvent comprises up to 85 percent by weight of the final composition. Preferably the amount of glycerol is up to 10 percent by weight of the final composition; most preferably about 5 percent by weight. Preferably the amount of polyethylene glycol (PEG) is up to 75 percent by weight of the final composition; most preferably about 60 to 70 percent by weight.

### Gel

The non-aqueous dentifrice composition according to the invention comprise a supporting (or gelling) polymer to solidify the solvent and actives.

The skilled person will be aware of many different types of supporting polymer for dentifrice use. Two non-limiting examples include a copolymer of a methyl vinyl ether and a maleic anhydride, such as Gantrez(R), and polyacrylic acid homopolymers, often referred to as Carbomers.

Suitably for the present invention the supporting (or gelling) polymer comprises a carboxyvinyl polymer such as a carbomer.

A carbomer comprises synthetic high molecular-weight cross-linked polymers of acrylic acid. The polymer chains formed of repeating units of acrylic acid may be cross-linked with, for example: allyl sucrose to provide a carbomer available commercially in one form as Carbopol^{™} 934; ethers of pentaerythritol to provide a carbomer available commercially in one form as Carbopol^{™} 974; or with divinyl glycol, available commercially in one form as Noveon^{™} AA-1. Carbopol^{™} polymers are manufactured by B.F. Goodrich Company.

In one embodiment the carboxyvinyl polymer comprises Carbopol^{™} 974.

The carboxyvinyl polymer may be present in the range of from about 0.1 to about 7.5 percent by weight of the non-aqueous composition. In one embodiment the carboxyvinyl polymer is present in an amount from about 0.2 to about 1.0 percent by weight of the composition.

### Abrasive

An abrasive may be required to aid the removal of detritus from the teeth, dentures and / or remainder of the oral cavity being cleaned. Clearly it will be appreciated that a degree of abrasiveness is required / advantageous in the removal of said detritus., However, it will also be appreciated that excessive abrasiveness can be equally detrimental: excessive wear on dental materials can be problematic. As such it will be appreciated that the use of an abrasive is a compromise of achieving adequate detritus removal without causing excessive /undesired wear on dental materials.

Suitable abrasives for use in the dentifrice composition include, for example, amorphous, gelled, precipitated or fumed silica, zinc orthophosphate, sodium bicarbonate (baking soda), plastic particles, alumina, calcium carbonate, calcium pyrophosphate, insoluble metaphosphates or mixtures thereof.

The silica abrasive may be a natural amorphous silica, for instance diatomaceous earth; or a synthetic amorphous silica such as a precipitated silica. By way of example, silica abrasives include those marketed under the following trade names Zeodent, Sident, Sorbosil or Tixosil by Huber, Degussa, Ineos and Rhodia respectively.

The silica abrasive may be a spherical, anhydrous, amorphous, silica gel particles having a pore volume of less than 0.1ml/g (as described in co-pending European Application Number 18782931.2). This abrasive preferably comprises non-fused spherical, anhydrous, amorphous, silica gel particles obtainable by a process that involves precipitation followed by calcination to remove water to produce a dense particulate silica material, and wherein said silica gel particles comprise:
a. a pore volume from 0.03 ml/g to less than 0.1 ml/g;
b. a mean particle size from 1 micro to 10 micro;
c. a BET surface area of 50 m²/g or less;
d. an oil absorption capacity of 20 to 50 ml/100 g; and
e. a water content of less than 0.2 weight percent.

Such silica gel particles have been found to effectively clean, polish and remove stains from the surface of teeth or dentures without a high degree of abrasion thereby reducing scratching and damage to the tooth or denture surface. Such compositions thereby provide superior cleaning, polishing, gentle stain removal and whitening of tooth surfaces or dentures.

Silica gel particles for use in the invention are available commercially from Asahi Glass SI-Tech. Co., Ltd., 13-1, Kitaminatomachi"Wakamatsu-ku, Fukuoka, 808-0027, Japan. and are sold under the brand name Sunspherc(a), for example Sunsphcre NP-30 and Sunsphcrc NP-100. A CAS1 for some types of silica gel particles of use in the invention is 7631-86-9.

Sunsphere(a) silica gel particles are derived from sodium silicate and contain no crystalline silica. They consist of spherical particles of almost identical size which do not cohere, and which provide a smooth texture.

Similar silica gel particles are available from Madhu Silica PVT. LTD under the brand name ( as an example) of MFIL-GS.

Most preferably where an abrasive silica is used it is desirable that the stability of hydrogen peroxide in presence of silica is preserved. It has been observed that spherical, anhydrous, amorphous, silica gel show high preservation of hydrogen peroxide. As such these are highly preferred to be used as an abrasive silica.

Suitably an abrasive is present in an amount up to 5 percent by weight of the total composition, for example from 0.1 to 4 percent by weight, for example from 0.15 to 3 percent, for example from 0.20 to 2 percent, for example from 0.25 to 1.5 percent, for example from 0.2 to 1 percent, for example from 0.25 to 1 percent by weight of the total composition.

Generally, an amount of abrasive suitable for use in the dentifrice composition of the present invention will be empirically determined to provide an acceptable level of cleaning and polishing, in accordance with the techniques well known in the art.

### Fluoride

The dentifrice compositions of the present invention preferably comprise a source of fluoride as required. Non-limiting examples of fluoride include stannous fluoride, sodium fluoride, sodium monofluorophosphate, potassium fluoride, ammonium fluoride, bis-(hydroxyethyl) amino-propyl-N-hydroxyethyloctadecylamine-dihydrofluoride and mixtures thereof. Preferably the fluoride required in the formula will be provided by stannous fluoride (SnF₂) and / or sodium fluoride (NaF).

The amount of fluoride allowed in dentifrice compositions is heavily controlled by regulatory bodies.

Suitably the composition comprises between 0.05 percent and 0.5 percent by weight of fluoride source. Preferably this equates to from 450ppm of fluoride to 1500ppm of fluoride. A preferred amount of fluoride can be about 900ppm, 1100ppm or about 1450ppm.

### Surfactant

The dentifrice composition according to the invention comprises at least one solvent, wherein the at least one surfactant comprises sodium lauryl sulphate.

The compositions of the present invention at least one surfactant may comprise a single surfactant. Alternatively, the at least one surfactant may comprise a mixture of a first surfactant and a second surfactant. Alternatively, the at least one surfactant may comprise a mixture of a first surfactant, a second surfactant and a third and/or further surfactants.

A suitable second surfactant for use in the compositions according to the invention belongs to the class of compounds known as betaines. Structurally, betaine compounds contain an anionic functional group such as a carboxylate functional group and a cationic functional group such as quaternary nitrogen functional group separated by a methylene moiety. They include n-alkyl betaines such as cetyl betaine and behenyl betaine, and n-alkylamido betaines such as cocoamidopropyl betaine.

In one embodiment the betaine is cocoamidopropyl betaine, commercially available under the trade name Tego Betain(R).

Suitably the betaine may be present in an amount ranging from about 0.05 to about 4 percent by weight of the dentifrice composition, for example from about 0.2 to about 2.0 percent, more preferably between about 0.3 to about 0.6 percent by weight of the non-aqueous composition.

Another surfactant for use in the dentifrice compositions according to the invention may be selected from a taurate based surfactant. Taurate surfactants useful in the present invention are salts of fatty acid amides of N-methyl taurine. They conform generally to the structural formula:

RC(O)N(CH₃)CH₂CH₂SO₃

Where RC(O)- represents a fatty acid radical and M represents sodium, potassium, ammonium or triethanolamine. Fatty acids having carbon chain lengths of from 10 to 20, including those derived from coconut, palm and tall oil are used. In one embodiment the fatty acid is derived from coconut. In one embodiment, sodium salts are used.

In one embodiment the taurate is sodium methyl cocyl taurate. This taurate surfactant is sold under the trademark by Adinol(R) CT by Croda.

The taurate surfactant may be present in an amount from about 0.1 to about 10 percent of the anhydrous dentifrice composition. In one embodiment the taurate surfactant is present in an amount from about 0.1 to about 5 percent by weight of the non-aqueous composition. In one embodiment the taurate surfactant is present in an amount from about 0.5 to about 2.0 percent by weight of the non-aqueous composition.

Another class of surfactants suitable for the present invention are alkyl sulphate surfactants of the following structural formula:

R¹OSO₃

R¹ represents a fatty alcohol moiety and M represents sodium, potassium, ammonium or triethanolamine. Fatty alcohols having carbon chain lengths of from about 10 to about 20, including those derived from coconut, palm oil and tall oil. In one embodiment, the fatty alcohol is lauryl alcohol. In one embodiment, a sodium salt is used.

The alkyl sulphate surfactant may be present in an amount from about 0.1 to about 10 percent of the nonaqueous composition. In one embodiment the alkyl sulphate surfactant may be present in an amount from about 0.1 to about 5 percent by weight of the non-aqueous dentifrice composition.

In one embodiment the alkyl sulphate surfactant is present in an amount from about 0.5 to about 2.0 percent by weight of the non-aqueous composition. In certain embodiments, the at least one surfactant comprises a first surfactant which is a C10-20 alkyl sulphate surfactant, and a second surfactant which consists of a betaine.

Preferably wherein the first surfactant is sodium lauryl sulphate (SLS) and the second surfactant is cocamidopropyl betaine.

Preferably the ratio of SLS and cocamidopropyl betaine is between 2:1 and 4:1 by weight. Most preferably the ratio of the SLS to cocamidoproyl betaine is about 3:1.

In a particularly preferred embodiment, SLS comprises between 1.0 and 1.4 percent by weight and the cocamidopropyl betaine comprises between 0.3 and 0.5 percent by weight of the non-aqueous dentifrice composition.

### Anti-Stain

The composition preferably comprises an anti-staining agent.

Preferably, where present, the anti-staining agent is present in an amount of up to 10 percent of the weight of the composition. Preferably the anti-staining agent is present in an amount of about 5 percent weight of the composition.

A preferred example of an anti-staining agent is anhydrous sodium tripolyphosphate (STP).

### Other

Compositions of the present invention may contain additional formulating agents such as flavouring agents, sweetening agents, opacifying or colouring agents and preservatives, selected from those conventionally used in an oral hygiene composition art for such purposes.

In general, the optional agents may be used in a minor amount or proportion of the overall formulation. By way of example, such components are usually present in from about 0.001 to about 5 percent by weight of the non-aqueous composition. In total such components may range from 0.1 to 25 percent by weight of the composition.

The dentifrice composition typically has a viscosity suitable for application to the oral cavity. The viscosity will vary depending on the type of dentifrice composition made and the ultimate use thereof. One of skill in the art can readily prepare compositions with suitable viscosities for use in the oral cavity from the teachings provided herein. Suitably a composition according to the invention may further comprise an inorganic thickening agent such as a thickening silica. Suitably, the thickening agent is a thickening silica, for example, a colloidal hydrated silica, available commercially for example as Sident 22S or Syloid 244FP.

Most preferably where a thickening silica is used it is desirable that the stability of hydrogen peroxide in presence of silica is preserved. It has been observed that silicas CSD (LBD) and CSD (HBD) show high preservation of hydrogen peroxide. As such these are highly preferred to be used as a thickening silica.

In one embodiment the thickening silica is present in the range of from about 0 to about 15 percent, suitably from about 5.0 to about 15.0 percent by weight of the non-aqueous composition.

The compositions according to the present invention may be prepared by admixing the ingredients in the appropriate relative amounts in any order that is convenient.

A dentifrice composition according to the present invention may be mildly acidic, neutral or mildly alkaline i.e. has a slurry pH in the range from 6.0 to 8.0 for example from pH 6.1 to 7.4, 6.2 to 7.3, or 6.2 to 7.2. In one embodiment the composition has a pH of about 6.2. In a further embodiment the composition has a pH of about 7.0. The pH referred to is that measured when the dentifrice composition is slurried with water in a 1:3 weight ratio of the composition to water. Suitably the slurry is prepared by slurring the dentifrice composition with water in a weight ratio of one part dentifrice composition and three parts distilled water. The pH is determined using a standard pH meter.

### Examples

The following formulations were prepared.

| **Material** | **Function** | **24/014** | **24/015** | **24/016** | **24/017** | **24/018** | **24/019** |
|---|---|---|---|---|---|---|---|
| | | %w/w | %w/w | %w/w | %w/w | %w/w | %w/w |
| Glycerol | Diluent | 5.0000 | 5.0000 | 5.0000 | 5.0000 | 5.0000 | 5.0000 |
| Macrogol 400 | Diluent | 67.750 | 67.9958 | 68.250 | 62.7000 | 63.2000 | 63.7000 |
| PVP-peroxide complex | Chemical whitening | 9.1000* | 9.1000* | 9.1000* | 13.6500 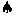 | 13.6500 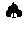 | 13.6500 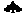 |
| Carbomer homopolymer | Gel | 0.9000 | 0.9000 | 0.9000 | 0.9000 | 0.9000 | 0.9000 |
| Aerosil 200 | Thickener | 30000 | 3.0000 | 3.0000 | 30000 | 3.0000 | 3.0000 |
| Spherical Silica | Abrasive | - | 0.2500 | - | - | 0.5000 | - |
| Alumina | Abrasive | 0.5000 | - | - | 1.0000 | 0.0000 | - |
| Potassium Nitrate | Anti-sensitivity | 5.0000 | 5.0000 | 5.0000 | 5.0000 | 5.0000 | 5.0000 |
| Sodium Fluoride | Anticaries | 0.2542 | 0.2542 | 0.2542 | 0.2542 | 0.2542 | 0.2542 |
| STP | Anti-stain | 5.0000 | 5.0000 | 5.0000 | 5.0000 | 5.0000 | 5.0000 |
| Sodium Lauryl sulphate | Surfactant | 2.0000 | 2.0000 | 2.0000 | 2.0000 | 2.0000 | 2.0000 |
| Flavour | Flavour | 1.0000 | 1.0000 | 1.0000 | 1.0000 | 1.0000 | 1.0000 |
| Sodium Saccharin | Sweetener | 0.5000 | 0.5000 | 0.5000 | 0.5000 | 0.5000 | 0.5000 |

| | | | | | | | |
|---|---|---|---|---|---|---|---|
| * equates to 2wt% hydrogen peroxide 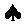 equates to 3wt% hydrogen peroxide | | | | | | | |

### Example 1: Relative Dentine Abrasivity Test (RDA) as a measure of Abrasivity on Bovine Enamel Samples

### Introduction

Dentifrice formulations above were assessed for their abrasivity (as determined by RDA).

### Methodology

The procedure used was the ADA recommended procedure for determination of dentifrice abrasivity as detailed in ISO11609 and ANSI/ADA Standard No. 130. The human dentin specimens (8) were placed in a neutron flux under the controlled conditions outlined by the ADA. The specimens were then mounted in methyl methacrylate so they would fit in a V-8 cross-brushing machine. The specimens were brushed for a 1500-stroke precondition run using a slurry consisting of 10g ISO/ADA reference material (calcium pyrophosphate powder; RDA standard grade, Odontex Inc., Lawrence, KS, USA) in 50 ml of a 0.5% CMC (carboxymethyl cellulose) glycerin solution. The brushes used were those specified by the ISO/ADA (Oral-B, P40, Procter and Gamble, Cincinnati, OH, USA) and brush tension was set to 150g.

Following the precondition run, the test was performed using 150g and 1500 strokes in a sandwich design in which each test material slurry (25g/40ml DI Water for dentifrices).

One (1.0) ml samples were taken, weighed (0.01g) and added to 4.5 ml of scintillation cocktail. The samples were mixed well and immediately put on the scintillation counter for radiation detection. Following counting, the net CPM values were divided by the weight of the sample to calculate a net CPM/gram of slurry. The net CPM/g of the pre and post ISO/ADA reference material for each test slurry were then calculated and averaged to use in the calculation of RDA (relative dentin abrasion) for the test material. The ISO/ADA material was assigned a value of 100 and its ratio to the test material was calculated. As of 16-Aug-1999, a new lot of the ISO/ADA reference material has been used.

This material is 3.6% less abrasive than old batches, thus requiring the addition of 3.6% to the reference material CPM.

This allows the direct comparison of this data to previous data.

### Example 2 Pellicle Cleaning Ratio (PCR) Testing of Dentifrices

### Specimen Preparation

Bovine, permanent, central incisors were cut to obtain labial enamel specimens approximately 8x8 mm2. The enamel specimens were then embedded in an auto-polymerizing methacrylate resin so that only the enamel surfaces were exposed. The enamel surfaces were then smoothed on a lapidary wheel and polished with flour of pumice and water, then sonicated to remove excess debris. They were then lightly etched (60 seconds in 0.12M HCl, 30 seconds in saturated NaCO3 and 60 seconds in 1.0% phytic acid) to expedite stain accumulation and adherence.

They were then placed on a rotating rod alternately exposing them to staining broth containing gastric mucin as a protein source and coffee, tea and FeCl3 6H2O as staining sources (i.e., 1.35g coffee, 1.35g tea, 0.02g Fe and 1.0 g mucin in 400 ml). After about 10 days the specimens normally develop stain so that the L* value is in the range of 30-38.

### Scoring and Set-Up

The amount of in vitro stain was determined spectrophotometrically (Minolta CM-26dG, Spectrophotometer) using only the L* value of the CIELAB color space scale. The area of the specimens scored was a 1/4 inch diameter circle in the center of the enamel sample. Specimens with L* values between 30-38 (30 being more darkly stained) were used. On the basis of these scores, the specimens were balanced into groups of N=16 specimens, with each group having the same average baseline score.

### Test Procedure

The specimens were then mounted on a mechanical V-8 cross-brushing machine equipped with soft nylon-filament (Oral-B 40 Indicator) toothbrushes. Toothbrushes were conditioned by running brushing machine for 1,000 strokes in deionized water. Tension on the enamel surface was adjusted to 150g. The dentifrices were prepared by mixing 25g of dentifrice with 40ml of DI Water. The ISO/ADA reference material was prepared by mixing 10g of material (calcium pyrophosphate powder; RDA standard grade, Odontex Inc., Lawrence, KS, USA) in 50 ml of a 0.5% CMC (carboxymethyl cellulose) glycerin solution. The specimens were brushed for 800 double strokes. To minimize mechanical variables, one specimen per group was brushed on each of the eight brushing heads. Each slurry was utilized to brush a single specimen. Following brushing, specimens were rinsed, blotted dry, and scored again for stain as previously described.

### Calculations

The difference between the pre- and post-brushing L* values was determined and the Mean (N=16), standard deviation (SD) and standard error (SEM) calculated. The cleaning ratio for the reference material group was assigned a PCR value of 100. The mean decrement for the reference group was divided into 100 to obtain a constant value to multiple times each individual test decrement within the study. The individual cleaning ratio of each specimen was then calculated (decrement X constant). The larger the value of the cleaning ratio, the greater the amount of stained pellicle removed in this test.

### Results and Statistical Analyses

The results are detailed in the attached tables and summarized in Table I below. The Mean (N=16), standard deviation (SD) and standard error (SEM) were calculated for each treatment group. Statistical analyses were performed with a one-way analysis of variance (ANOVA) model using Sigma Plot (14.5) Software. Since the ANOVA indicated significant differences, the individual means were analyzed by Student-Newman-Keuls (SNK) pairwise analysis. Significance of all analyses was determined at P < 0.050.

### Results

The RDA and PCR results are tabulated below.

| **Sample** | **Mean RDA** | **Mean PCR** |
|---|---|---|
| 24/014 | 34.11 | 101.5 |
| 24/015 | 30.21 | 143.9 |
| 24/016 | Not tested | 79.9 |
| 24/017 | 29.11 | 114.6 |
| 24/018 | 41.08 | 163.6 |
| 24/019 | Not tested | 101.3 |

### Conclusions

### RDA

Test sample 24/018 (41.08 ± 1.75) was significantly more abrasive (P ≤ 0.003) to dentin compared to test samples NPD 24/014 (34.11 ± 1.46), NPD 24/015 (30.21 ± 1.96) and 24/017 (29.11 ± 0.97), while these test samples did not differ significantly (P ≥ 0.078) in relative dentin abrasivity in this in vitro assessment.

All test samples in this in vitro assessment exhibited RDA values within the acceptable limit (RDA ≤ 250) for dentifrice dentin abrasivity as defined by ISO and ANSI/ADA standards.

### PCR

Test sample 24/018 (PCR = 163.6 ± 4.5 | Mean ± SEM) exhibited a significantly greater (P ≤ 0.001) stained pellicle cleaning efficacy compared to all other test samples in this in vitro assessment. Test sample 24/016 (PCR = 79.9 ±4.2) exhibited significantly less (P ≤ 0.002) stained pellicle cleaning efficacy compared to all other test samples in this in vitro assessment. The remaining test samples in this in vitro assessment exhibited Mean (N=16) pellicle cleaning ratio (PCR) values ranging from PCR = 101.3 (24/019) to PCR = 143.9 (24/015).
the silica gel particles of compositions 24/015 and 24/018 have been found to effectively clean, polish and remove stains from the surface of teeth or dentures without a high degree of abrasion thereby reducing scratching and damage to the tooth or denture surface. Such compositions thereby provide superior cleaning, polishing, gentle stain removal and whitening of tooth surfaces or dentures.

The examples all showed superior whitening and anti-sensitivity properties.

## Claims

1. A dentifrice composition comprising:
e) cross-linked polyvinylpyrrolidone-hydrogen peroxide complex (cPVP-H₂O₂ complex),
f) sodium tripolyphosphate (STP),
g) at least one solvent, wherein the at least one solvent comprises sodium lauryl sulphate, and
h) at least one desensitising agent, wherein the at least one desensitizing agent comprises potassium nitrate.

2. The dentifrice composition according to claim 1, wherein the amount of cPVP-H₂O₂ complex is selected to provide from about 0.01 weight percent to about 6 weight percent hydrogen peroxide to the dentifrice composition.

3. The dentifrice composition according to claim 1 or 2, wherein the potassium nitrate is present in an amount of 0.1 to 10 % by weight of the composition, preferably 4-6 % by weight.

4. The dentifrice composition according to any of the preceding claims wherein the amount of STP is present in an amount of 2 to 8 % by weight of the composition, preferably 4-6 % by weight

5. The dentifrice composition according to any of the preceding claims wherein the sodium lauryl sulphate is present in an amount equal to 1-3% by weight of the composition.

6. The dentifrice composition according to any of the preceding claims, wherein the composition comprises a single phase non-aqueous composition comprising at least one solvent, wherein the solvent comprises 55-80% by weight.

7. The dentifrice composition according to claim 6, wherein the at least one solvent comprises glycerol, polyethylene glycol (PEG) or a mixture thereof.

8. The dentifrice composition according to any of the preceding claims, wherein the composition comprises an abrasive, wherein the abrasive is present up to 10 % by weight, preferably between 0.25 and 5 % by weight.

9. The dentifrice composition according to claim 7 wherein the abrasive is selected from the group comprising amorphous, gelled, precipitated or fumed silica, zinc orthophosphate, sodium bicarbonate (baking soda), plastic particles, alumina, hydrated alumina, calcium carbonate, calcium pyrophosphate, insoluble metaphosphates or mixtures thereof.

10. The dentifrice composition according to any of the preceding claims, wherein the composition comprises a source of fluoride, and wherein the source of fluoride is selected from the group comprising stannous fluoride, sodium fluoride, sodium monofluorophosphate, potassium fluoride, ammonium fluoride, bis-(hydroxyethyl) amino-propyl-N-hydroxyethyloctadecylamine-dihydrofluoride and mixtures thereof. Preferably the fluoride required in the formula will be provided by stannous fluoride (SnF₂) and / or sodium fluoride (NaF).

11. The dentifrice composition according to any of the previous claims wherein the composition comprises:
Glycerol - 3 - 7% by weight,
PEG - 55 - 70% by weight,
PVP-peroxide complex - 1.0 - 4.0 % by weight (of H₂O₂),
STP - 4-6% by weight,
Potassium nitrate - 4-6 % by weight
Carbomer - 0.5-1.5 % by weight,
Abrasive - 0.25-5 % by weight,
Sodium lauryl sulphate - 1-4% by weight,
Fumed silica - 0.1- 2% by weight, and
a source of fluoride.

12. The dentifrice composition according to any of the previous claims wherein the composition comprises:
Glycerol - 3 - 7% by weight,
PEG - 60 - 70% by weight,
PVP-peroxide complex - 1.5 - 3.5 % by weight (of H₂O₂),
STP - 4-6% by weight,
Potassium nitrate - 4-6% by weight
Carbomer - 0.8-1.2 % by weight,
Alumina - 0.5 - 1.5 % by weight,
Sodium lauryl sulphate - 1-4% by weight,
Fumed silica - 0.1- 2% by weight, and
a source of fluoride, wherein the source of fluoride is NaF.

13. The dentifrice composition according to any of the previous claims wherein the composition comprises:
Glycerol - 3 - 7 % by weight.
PEG - 60 - 70 % by weight,
PVP-peroxide complex - 1.5 - 3.5 % by weight (of H₂O₂),
STP - 4 - 6 % by weight,
Potassium nitrate - 4 - 6 % by weight
Carbomer - 0.5 - 1.5 % by weight,
Spherical silica - 0.25 -1 % by weight,
Sodium lauryl sulphate - 1-4 % by weight,
Fumed silica - 0.1- 2 % by weight, and
a source of fluoride, and wherein the source of fluoride is NaF.

14. A use of the dentifrice composition according to any of claims 1 to 13 in combination with a toothbrush to clean teeth.

15. A method for tooth whitening including applying the tooth composition of to any of claims 1 to 13 to at least one tooth.
